(19) Europäisches Patentamt
European Patent Office
Office européen des brevets

(11) **EP 2 975 424 A1**

(12) **EUROPEAN PATENT APPLICATION**

(43) Date of publication:
**20.01.2016 Bulletin 2016/03**

(51) Int Cl.:
**G01R 33/20** (2006.01)  **A61B 5/055** (2006.01)
**G01R 33/48** (2006.01)

(21) Application number: **14177733.4**

(22) Date of filing: **18.07.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Universität Bern
3012 Bern (CH)**

(72) Inventor: **The designation of the inventor has not
yet been filed**

(74) Representative: **Schmauder & Partner AG
Patent- & Markenanwälte VSP
Zwängiweg 7
8038 Zürich (CH)**

(54) **Magnetic resonance imaging method for detecting neuronal activity in a subject's brain**

(57)      A magnetic resonance (MR) imaging method for detecting neuronal activity in a subject's brain comprises performing a first MR imaging sequence part (a) comprising a preparatory spin-locking pulse sequence part followed by a GE-EPI acquisition pulse sequence part, followed by a second MR imaging sequence part (b) comprising the same acquisition pulse sequence part without a preceding spin-locking pulse sequence part, whereafter steps (a) and (b) are repeated for a predetermined number of instances, each time using a different initial spin-lock phase. In order to detect neuronal activity in a subject's brain the MR imaging method is carried out with the subject's brain positioned in the MR imaging system. Difference MR data are obtained by subtracting said second MR data from said first MR data, wherein zones of comparatively high brightness are indicative of high-frequency neuronal currents.

Fig. 1

**Description**

Field of the Invention

The present invention generally relates to a magnetic resonance (MR) imaging method, particularly for detecting neuronal activity in a subject's brain. Moreover, the invention relates to a method of detecting neuronal activity in a subject's brain, and to a computer program product for causing a computer system to control the execution of the method.

Background of the Invention

The direct detection of neuronal activity using MR imaging techniques has been pursued for over a decade and the progress has recently been reviewed (Bandettini et al., 2005). Most of the proposed techniques are based on the detection of phase shifts or dephasing caused by the extremely small changes in the local B0 field induced by local neuronal currents. The phase shift or dephasing of the detected MR magnetization accumulates during the echo time (TE) period of either a gradient echo or spin echo sequence. The phantom findings are generally encouraging, with detectable local magnetic fields as small as 0.2 nT (Bodurka and Bandettini, 2002) and current dipoles as small as 6.3 nAm (Konn et al., 2003), but the in vivo literature contains just a few positive findings (Chow et al., 2006; Xiong et al., 2003), but mostly negative findings (Chu et al., 2004), including a failure to reproduce the paradigm of Xiong et al. (Parkes et al., 2007). Understanding the microscopic spatial distribution and time course of the neuronal currents is critical to assessing the potential ability of these MR-based methods to detect neuronal activation. The phase-sensitive portion of the MR sequence must be properly timed relative to the transient or oscillating neuronal current to ensure that cancellation of the induced phase during a biphasic or multi-phasic current is minimized.

Conventional methods using T2 and T2* are only sensitive to the component of the local field along the applied external B0 field. The spatial distribution of the current within the voxel can also lead to cancellation of the phase shift induced in the MR signal. Even a DC current in a small, straight wire running through the center of the voxel will not induce a net phase shift in the MR signal in that voxel; the current must be offset from the voxel center to produce a net phase accrual (Bandettini et al., 2005). This arises since the phase is sensitive to the sign of the magnetic field and thus the integral of the phase shift over the voxel is zero for this symmetric case.

Recently, the use of ultra-low-field MRI has been proposed to detect neuronal currents (Kraus, 2006; Kraus et al., 2008). An extremely low B0 measurement field allows resonant interactions between the neuronal currents and spin magnetization if $\gamma$B0 is brought into the frequency range of the neuronal currents. Although their first measurements were at significantly higher fields, Kraus and colleagues proposed static measurement fields weaker than 0.025 G using a SQUID magnetometer to detect the MR signals at a Larmor frequency of less than 100 Hz (Matlachov et al., 2004; Volegov et al., 2004). In this scenario, the transient neuronal fields can possibly act as resonant excitation pulse providing the initial excitation of the proton magnetization. Thus, the neuronal current supplies the local B1 field for excitation and no transverse magnetization should be detected in locations without neuronal currents.

In view of various shortcomings, but also of uncertainties about practical implementation of presently attempted methods of direct detection of neuronal activity, it is an object of the present invention to provide an improved MR imaging method, particularly for detecting neuronal activity in a subject's brain.

Further objects of the invention are a method of detecting neuronal activity in a subject's brain, particularly in relation to epileptic events, and a computer program product for execution of the detection method.

Summary of the Invention

According to one aspect of the invention, there is provided a magnetic resonance (MR) imaging method, particularly for detecting neuronal activity in a subject's brain, the method comprising:

a) performing a first MR imaging sequence part with a MR imaging system having a static magnetic field in z-direction, said first sequence part comprising:

- a preparatory spin-locking pulse sequence part comprising an initial spin-tipping pulse followed by a spin-lock pulse followed by a final spin-tipping pulse, followed by spoiler gradient pulses for removing any remaining transverse magnetization;
wherein said initial spin-tipping pulse is a 90° pulse in y direction and said final spin-tipping pulse is a 90° pulse in -y direction, or vice versa;
wherein said spin-lock pulse is a rectangular pulse in x direction consisting of two phase-alternating halves and having an amplitude $B_{1lock}$, a spin-lock pulse duration TSL and an initial spin-lock phase $\varphi_{1SL}$;
- an acquisition pulse sequence part to acquire first MR data, wherein said acquisition pulse sequence part is an echo planar imaging sequence;

b) performing a second MR imaging sequence part with said MR imaging system, said second sequence part comprising said acquisition pulse sequence part to acquire second MR data; and

c) repeating steps a) and b) for a predetermined number of instances, wherein the initial spin-lock phase $\varphi_{1SL}$ is increased by a predetermined phase increment $\Delta\varphi_{1SL}$, thereby obtaining multiple sets of first and second MR data.

The MR imaging system is of a generally known type and comprises means for generating a static, i.e. stationary homogeneous magnetic field in z-direction, means for exciting RF pulses, means for generating magnetic field gradients, means for detecting and sampling magnetic resonance signals obtained with said means in an object or subject which is situated in the homogeneous field, control means for controlling said means, image reconstruction means, and controller means programmed to run any predetermined MR imaging sequences.

In the present context, the term "MR imaging sequence" shall be understood as a temporal sequence of steps as generally known in the field of MR imaging, including RF pulses of various shapes and gradient field pulses. Although the term "sequence" is often understood as meaning the entirety of individual steps needed to carry out a complete measurement or image acquisition, in some situations it is also used to denote just a subset of steps that are repeated either identically or with some modification for a plurality of times, so that it will be more appropriate to speak of a "sequence portion" or "sequence part". In the present context, the term "sequence part" will be used to denote any specific set of steps that are carried out as part of what could be understood as the "overall sequence" of steps needed to complete the claimed method.

The method of the present invention relies on a spin-locking mechanism (Abragam, 1961) to effectively lower the Larmor frequency in a high-field MR system down into the acoustic range. It is based on a double irradiation rotary saturation technique. During a spin-lock, the magnetization is locked in the rotating frame by an RF field, the so-called spin-lock field B1lock. The effective resonance frequency in the rotating frame during the spin-lock condition is very close to $\omega_{1lock} = \gamma B_{1lock}$. The desired Larmor frequency in the rotating frame can be chosen by setting the RF power of the spin-lock pulse.

The method of the present invention is based on an overall sequence comprising two sub-sequences called here "first MR imaging sequence part" and "second MR imaging sequence part" leading to the acquisition of first MR data and second MR data, respectively. In simple terms, the first MR imaging sequence part comprises a preparatory pulse sequence part followed by an acquisition pulse sequence part, whereas the second MR imaging sequence part merely comprises the acquisition pulse sequence part.

The first MR imaging sequence part corresponds to a pulse sequence for rapid in vivo spin-locked MRI that has been previously described by Borthakur et al., 2006. It generally comprises a preparatory spin-locking pulse sequence part and an acquisition pulse sequence part.

The preparatory spin-locking pulse sequence part comprises an initial spin-tipping pulse configured as a 90° pulse in y direction, which flips the overall magnetization into the xy-plane. Thereafter, a spin-lock pulse configured as a rectangular pulse in x direction and having an amplitude $B_{1lock}$, a spin-lock pulse duration TSL and an initial spin-lock phase $\varphi_{1SL}$ is applied. As explained in more detail further below, the Larmor frequency during the spin-lock pulse is shifted from RF to a substantially lower frequency and thereby allows for sensitive acquisition of signals with components in a low frequency range. After the spin-locking period, a final spin-tipping pulse configured as a 90° pulse in -y direction is applied to return the overall magnetization in z direction. It will be understood that equivalent results could be obtained by switching the sign of the two 90° pulses. In order to remove any transverse magnetization, a series of spoiler gradient pulses are applied after the final spin-tipping pulse. The spin-lock pulse consists of two phase-alternating halves, i.e. the second half is phase-shifted by $\pm90°$ from the phase of the first half, which allows reducing the undesirable effects of B1 field inhomogeneity.

The acquisition pulse sequence part is an echo planar imaging (EPI) sequence. The same acquisition pulse sequence part is used in the first MR imaging sequence part a) and in the second MR imaging sequence part b).

The above mentioned first and second MR imaging sequence parts form the building blocks of an "on-off-spin-locking scheme". This scheme is repeated for a predetermined number of instances, whereby the initial spin-lock phase $\varphi_{1SL}$ is increased by a predetermined phase increment $\Delta\varphi_{1SL}$ so as to obtain multiple sets of first and second MR data. For the avoidance of doubt, it shall be understood that $\Delta\varphi_{1SL}$ could be positive or negative.

The entire data acquisition process just described can itself be repeated for signal averaging purposes so as to obtain the required data quality.

The multiple sets of first and second MR data are generally stored for subsequent data processing, particularly for the purpose of image reconstruction.

Advantageous embodiments of the invention are defined in the dependent claims and/or are described herein below.

According to the invention, the acquisition pulse sequence part is an echo planar imaging sequence, for which various embodiments are known.

According to an advantageous embodiment (claim 2), the echo planar imaging sequence is a gradient echo planar imaging sequence (GRE). This name is related to the fact that in GRE sequences the 180° echo-forming pulse is omitted and the magnetization is refocused solely by appropriately configured gradient pulses.

According to an advantageous embodiment (claim 3), the spin-lock pulse has an amplitude $B_{1lock}$ corresponding to a Larmor frequency of 100 to 200 Hz, preferably about 120 Hz. This has been found to be particularly suitable for the detection and concurrent imaging of neuronal currents in a subject's brain.

According to a further embodiment (claim 4), the spin-lock pulse duration TSL is 50 to 200 ms, preferably about 100 ms. This has been found to be advantageous in terms of signal quality and acquisition time.

According to a further embodiment (claim 5), the phase increment $\Delta\varphi_{1SL}$ is 40° to 60°, preferably about 50°. This so-called "phase cycling" allows for detection of dipoles of different orientations.

According to another aspect of the invention, there is provided a method of detecting neuronal activity in a subject's brain, comprising:

a) carrying out the MR imaging method of the present invention with the subject's brain positioned in the MR imaging system;
b) obtaining difference MR data by subtracting said second MR data from said first MR data; and
c) generating an image from said difference MR data, wherein zones of comparatively high brightness are indicative of high-frequency neuronal currents.

In contrast to other methods, most notably the so-called BOLD (Blood Oxygenation Level Dependent) method, the detection method of the present invention provides a result that is directly related to certain neuronal current components in a subject's brain. It will thus be denoted here as a method of neuronal current imaging and henceforth abbreviated as "NCI". As will be discussed further below, if neuronal events in the subject's brain lead to magnetic pulses that are collinear with the spin-locking axes, a resonance condition is fulfilled and a pre-saturation of magnetization occurs during the spin-locking pulse sequence part. Thereafter, the signal acquired by means of the acquisition pulse sequence part will be lower than the signal acquired by means of the same acquisition pulse sequence part without preceding spin-lock. When epileptic events lead to magnetic pulses having a modulation frequency clearly higher than the spin-lock frequency, the resonance condition is not fulfilled and thus the spin-locked related signal will be similar to that without spin-lock.

The term "high-frequency neuronal currents" refers to frequency components in the acoustic range, particularly in the range of 100 to 200 Hz, preferably about 120 Hz (claim 3).

According to one embodiment (claim 7), the image is represented as a z-score map obtained by a raw image reconstructed from said difference MR data. The use of z-score maps is known in MRI, but it turns out to be particularly helpful in improving the quality of images obtained according to the present invention.

According to a further embodiment (claim 8), the z-score map includes a threshold of 50 to 70%, preferably about 60%. The latter threshold value has been found suitable for acquiring NCI related images over a broad range of experimental conditions.

According to a further aspect of the invention, there is provided a computer program product comprising program code means stored on a computer readable medium for causing a computer system to control the execution of the method of detecting neuronal activity in a subject's brain when the computer program product is run on the computer system. The computer readable medium on which said program code means are stored are any suitable data storage means such as transportable storage media, e.g. DVD or memory sticks, or storage media permanently installed in a computer or processor linked to a MRI apparatus, or remote storage media accessible via a computer network or cloud.

Brief description of the drawings

The above mentioned and other features and objects of this invention and the manner of achieving them will become more apparent and this invention itself will be better understood by reference to the following description of various embodiments of this invention taken in conjunction with the accompanying drawings, wherein:

Fig. 1     is a diagram of an MR sequence according to one embodiment;

Fig. 2     a) shows a sagittal image of a subject's head with exemplary vectors B(r,t) and J(r,t); b) illustrates a spin-lock experiment for situations "in resonance" and "not in resonance", respectively;

Fig. 3     shows a simulation of a NCI sequence signal simulation;

Fig. 4    shows a simulation of B0/B1 effects based on the Bloch equations, assuming initial Gaussian B0/B1 distributions for two different lengths of the spin-lock pulse, namely a) TSL = 50 ms and b) TSL = 100 ms;

Fig. 5    shows electroencephalography (EEG) (left) and NCI data (right) as transverse cranial sections for patients with epilepsy, with SOZ denoting seizure onset zones; and

Fig. 6    shows a comparison of a) NCI phase and b) NCI magnitude with overlaid z-scores.

Detailed description of the invention

*General remarks*

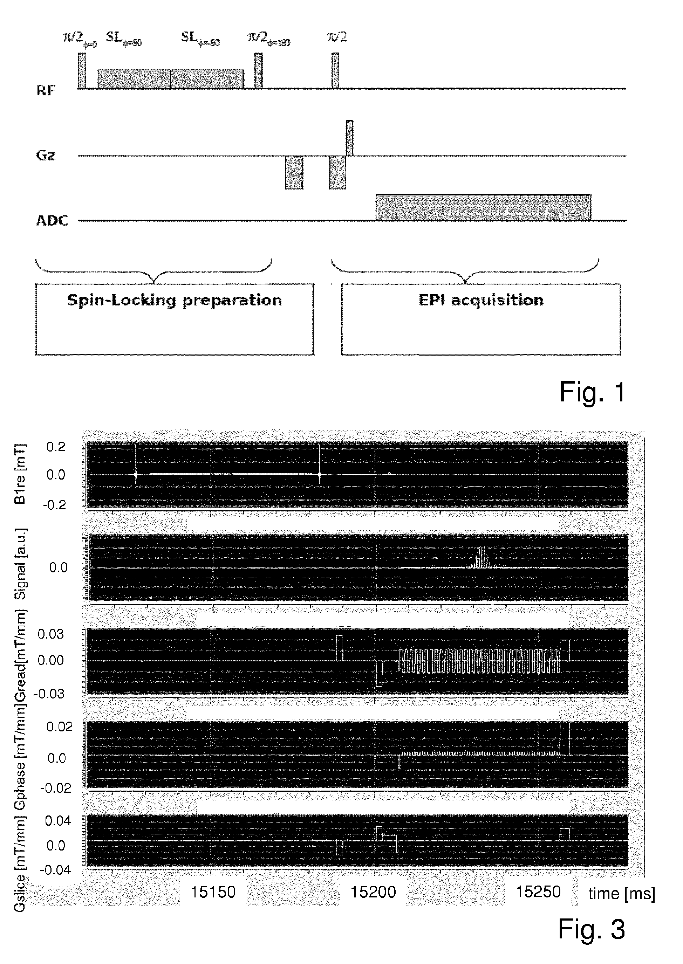

The present method relies on a dual scheme using both spin-locking and usual Gradient Recalled Echo (GRE), as employed e.g. for BOLD measurements, in order to generate a difference of the signal contributions afterwards. As shown in Fig. 1, the spin-lock (SL) part consists of a 90° hard pulse in y direction, immediately followed by a rectangular spin-lock pulse in x direction, which remains on for the duration TSL. The spin-lock period is followed by a hard 90° pulse in -y direction which returns the spin-locked magnetization to the longitudinal axis for detection by the gradient echo EPI sequence. All remaining transverse magnetization is spoiled by immediately following spoiler gradients. The entire spin-lock preparation is non-selective. The initial nonselective 90° pulse along the x axis nutates the magnetization into the transverse plane along the y axis. The magnetization vector is then spin-locked with amplitude B1 for a duration of TSL (50-100 ms) with an on-resonance spin-lock pulse applied in line with the magnetization vector along the y axis. The spin-lock pulse is split into two phase-alternating halves along the positive and negative y axes to reduce the effects of B1 inhomogeneity. At the end of the spin-lock pulse, the magnetization vector is nutated back into the longitudinal axis using a nonselective 90° pulse along the x axis. This pulse cluster is pre-encoded before a conventional echo planar imaging (EPI) sequence to create a single-slice spin-lock pulse sequence. In the measurements described henceforth, the EPI sequence was a gradient echo (GRE) planar imaging sequence.

In our spin-locking setting the spins are sensitized to neuronal magnetic fields oscillating at the Larmor frequency in the rotating frame, where $\gamma$ $B_{1lock}$ is fixed to e.g. 120 Hz, as set by the applied $B_{1lock}$. Oscillating neuronal currents with spectral power at $\gamma B_{1lock}$ are then capable of producing rotary saturation of the spin-locked magnetization. Thus, the neuronal currents produce a resonant saturation effect on the MR signal in the rotating frame during the spin-lock state, and the spin-lock state can be tuned to be sensitive to a frequency of interest by adjusting $\gamma B_{1lock}$. In practice, $\gamma B_{1lock}$ is chosen to match the expected frequency of the neuronal oscillations to sensitize the sequence to these frequencies. Thus the neuronal currents induced by the stimulus create the rotary saturation effect.

In our sequence we performed a stepping procedure of the initial SL-pulse phase in multiples of $\Delta\varphi_{1SL} = 50°$ in order to detect dipoles with different spatial orientation and to minimize dependencies among different scans. The timing adopted for data acquisition of a given volume element is as follows: one volume with spin-locking, one without, one with, and so on. This enables to minimize the global (BOLD) effects during post-processing by pairwise subtraction of the signals. For averaging purposes the number of acquisitions (repetitions) ranges from 150 (TSL = 100 ms) to 200 (TSL = 50 ms) resulting in a measurement time of 13 min. Standardized Z-score maps are calculated along the temporal dimension.

*Spin-Locking Theory*

When a strong RF field is applied to a system of nuclear spins, except at exact resonance where it is small, the steady state precessing magnetization is very nearly parallel to the effective field. This is true in solids according to the assumption of spin temperature in the rotating frame; it is also true in liquids: for $\gamma^2 H_1^2 T_1 T_2 \gg 1$ and at a distance $H_0 - H^* = H_1 \cot\Theta$ from resonance, the steady state solutions of the Bloch equations can be rewritten as:

$$M_x/M_0 = \tan\Theta \, / \, [1 + ((T_1/T_2)\tan^2\Theta)]$$

$$M_y/M_0 = \tan^2\Theta/(\gamma H_1 T_2[1 + (T_1/T_2)\tan^2\Theta]) \ll 1, \text{ if } \gamma H_1 T_2 \gg 1$$

$$M_z/M_0 = 1 \, / \, [1 + (T_1/T_2)\tan^2\Theta] \tag{1}$$

From this it is apparent that the steady state magnetization Mp, with its three components in the rotating frame given by (1), is very nearly parallel to the effective field He. In the rotating frame R the magnetization Mp appears as an equilibrium d.c. magnetization aligned along a d.c. field He and possessing a Larmor frequency $\omega_e = -\gamma H_e$.

A second field $H_a$ rotating in a plane perpendicular to He with an angular velocity $\omega_a$ with respect to the frame R will strongly affect the magnetization and make its component $M_z$ along the effective field depart from its steady state value Mp, if $\omega_a$ is in the neighborhood of $\omega_e$. Such a field is easily produced by means of an oscillating field parallel to the d.c. field $H_0$. This field is decomposed into two oscillating fields, the first parallel to He and the second in the plane perpendicular to $H_e$. The latter is decomposed in the usual way into two rotating components. If $H'_a$ is the amplitude of the oscillating field, the rotating component of interest has an amplitude $H_a = 0.5H'_a \sin\Theta$. The correspondence between such a rotary resonance experiment and an ordinary one is as follows:

$$H_e -> H_0, \quad M_p -> M_0, \quad H_a -> H_1, \quad M_p - M_z -> M_0 - M_z$$

Just as in an ordinary resonance experiment the saturation makes the longitudinal magnetization depart from its equilibrium value $M_0$, so in rotary saturation the component $M_z$ along the effective field He departs from its equilibrium value Mp.

The distinction between ordinary and rotary saturations is in the detection. In ordinary resonance the absorption of RF energy by the spin system is detected directly. In rotary resonance, for the fields $H_1$ obtainable in practice, the frequency $\omega_e = -\gamma H_e$ falls in the acoustic range and the corresponding absorption of energy is exceedingly small. The occurrence of rotary resonance for $\omega_a = \omega_e$ is detected by the change it induces in $M_z$, which causes a change of the dispersion signal at the high frequency $\omega \sim -\gamma H_0$. The analogue of that kind of detection in an ordinary resonance experiment would be the observation of a change in the static magnetization, observed, for instance, by conventional susceptibility measurements, when the spin resonance is saturated.

At exact resonance the magnetization $M_p = 2\chi' H_1$ vanishes. However, its derivative with respect to $H_0$ is a maximum and equal to $M_0 T_2/H_1 T_1$. If the d.c. field $H_0$ is modulated to either side of the resonant value $H^* = -\omega/\gamma$ under slow-passage conditions, that is, at a frequency $\Omega << 1/T_1, 1/T_2, \gamma H_1$, for each $H_0 \neq H^*$, the value $Mp(H_0)$ of the quasi steady-state magnetization will be

$$M_p(H_0) = (H_0 - H^*)M_0 T_2/(H_1 T_1).$$

The audio-frequency field $H_a$ at a frequency $\omega_a = -\gamma H_1 = -\gamma[(H_0-H^*)^2 + H_1^2]^{1/2}$ will reduce $M_p(H_0)$ to smaller value $M_z(H_0) < M_p(H_0)$, and the dispersion derivative signal will be decreased.

If the amplitude of the audio field and that of the modulation of the d.c. field are not both much smaller than $H_1$, the rotary saturation curve will be asymmetrical, and its maximum will be shifted towards values of $|\omega_a|$ higher then $|\gamma H_1|$. If $H_a/H_1$ is not very small this is caused by the Bloch-Siegert shift (Bloch and Siegert, 1940), and if $|(H_0-H^*)/H_i|$ is not very small, by the fact that during the modulation cycle $H_e > H_1$, both effects being quadratic with respect to $|H_a/H_1|$ or $|(H_0-H^*)/H_1|$.

A more quantitative justification of rotary saturation is as follows. In the rotating frame and in the presence of an audio field $H_a(t)$ of amplitude $H_a$, rotating at a frequency $\omega_a = \omega_e = -\gamma H_e$, the equation of motion of the magnetization can be written (assuming $T_1 = T_2$ for simplicity)

$$dM/dt = \gamma M \times (H_e + H_a(t)) - (M-M_0)/T_1$$

$$= \gamma M \times (H_e + H_a(t)) - (M-M_p)/T_1 - (M_p-M_0)/T_1 \qquad (2)$$

Since Mp is a solution of $\gamma M_p \times H_e - (M_p-M_0)/T_1 = 0$, the vector $(M_p-M_0)/T_1$ is perpendicular to He. If we transform (2) to the doubly rotating frame, rotating at $\omega_a = \omega_e = -\gamma H_e$ around the vector He very nearly parallel to Mp, it becomes

$$\delta M/\delta t = \gamma M \times [H_e(1 - (\omega_a/\omega_e)) + H_a] - (M-M_p)/T_1 + \text{time-dependent terms} \qquad (3)$$

The time-dependent terms result from the vector $(M_p-M_0)/T_1$, which in the doubly-rotating frame is seen as rotating at the frequency $\omega a$. If one assumes that the effect of these terms can be neglected, the equation (3) is the exact analogue of an ordinary Bloch equation in a simply rotating frame, with a d.c. field He and an RF field $H_a$, the relaxation taking place towards the equilibrium d.c. magnetization $M_p$. The treatment of rotary resonance the becomes formally identical with that of ordinary resonance.

During a spin-lock, the magnetization is locked in the rotating frame by an RF field, the so-called spin-lock field $B_{1lock}$. The effective resonance frequency in the rotating frame during the spin-lock condition is very close to $\omega_{1lock} = \gamma B_{1lock}$, deviating only by a potential Bloch-Siegert shift. This latter small shift in resonance frequency results from the counter-rotating field component of a linearly polarized time varying field. The Larmor frequency in the rotating frame can be chosen by setting the RF power of the spin-lock pulse.

Redfield described the saturation of the spin-locked magnetization with an external coil and audio-frequency source as "rotary saturation" (Redfield, 1955). Redfield analyzed rotary saturation in solids using $B_{1lock}$ fields large compared to the local fields. They derived a steady state magnetization change in the detected spin-locked magnetization $M_\rho$ compared to its initial value $M_0$ for the solid state which is given by:

$$(M_0 - M_\rho)/M_0 = \gamma^2 B^2_{\text{rotarysat}} \pi T_{1\rho} f(\omega_{1\text{lock}})$$

where, $B_{\text{rotarysat}}$ is the applied rotary saturation field (a function hopefully carried out by the oscillating neuronal fields), $T_{1\rho}$ is the spin-lattice relaxation in the spin-locked state and $f(\omega_{1lock})$ is the Lorentzian shape function which has a value of 1 s on resonance, when $\gamma B_{1lock} = \gamma B_{\text{rotarysat}}$ (Abragam, 1961). In order to model the effect in liquids and for transitory applications of a rotary saturation field, we model the rotary saturation effect as a coherent rotation away from the B1lock direction by the rotary saturation field (i.e., the neuronal field). The equations of motion for the magnetization in the spin-lock state are formulated in the doubly rotating frame as

$$\frac{dM}{dt} = \gamma M \times \left[ H_{\text{eff}} \left( 1 - \frac{\omega_{\text{rotarysat}}}{\gamma |H_{\text{eff}}|} \right) + B_{\text{rotarysat}} \right] - R(M - M_\rho),$$

$$R = \begin{pmatrix} \dfrac{1}{T_2^*} & 0 & 0 \\ 0 & \dfrac{1}{T_2^*} & 0 \\ 0 & 0 & \dfrac{1}{T_{1\rho}} \end{pmatrix},$$

where $H_{\text{eff}}$ is the effective field formed from the applied $B_{1lock}$ and the off-resonance pseudo-field $\Delta\omega/\gamma$, $M_\rho$ denotes the equilibrium magnetization in the rotating frame, which is very small since $B_{1lock}$ is small. Here $H_{\text{eff}}$ is the relevant axis toward which the magnetization is at equilibrium, and is not the static magnetic field direction. If both the spin-lock RF and the Rotary Saturation field $B_{\text{rotary-sat}}$ is on resonance, then $H_{\text{eff}}$ is $B_{1lock}$. $B_{\text{rotarysat}}$ is the audio-frequency field applied orthogonal to the spin-lock axis (here $B_{1lock}$ is taken to be along the x axis in the singly rotating frame). To preserve the analogy to the singly rotating from, the axes of the doubly rotating frame are renamed so that the z axis is the equilibrium direction. Thus in the doubly rotating frame, the z axis is the direction $B_{1lock}$. Then $B_{\text{rotarysat}}$ must be applied orthogonal to $B_{1lock}$ to produce a rotation. The magnetization relaxes along $H_{\text{eff}}$ with the time constant $T_{1\rho}$, and in the other two directions with the time constant $T_2^*$.

The current density J(r) produced by neuronal activity can be described by two additive components: a primary $J^P$ and a volume current density $J^V$: $J(r,t) = J^P(r,t) + \sigma(r)E(r,t)$ (primary current density $J^P(r)$, volume/return current density $J^V(r,t) = \sigma(r)E(r,t)$, electric field strength E, conductivity $\sigma$). The primary active component $J^P$ is mainly inside or in the vicinity of a cell, whereas the volume part $J^V$ is passive and the result of the macroscopic electric field on charge carriers in the conducting medium. Neuronal current imaging (NCI) seems to be sensitive to both, $J^V$ and $J^P$. The relation of the current density with the magnetic field density B comes from Biot-Savart $B(r,t) = (\mu_0/4\pi) \int J(r',t) \times R/R^3 \, dv'$ - therefore there is no simple correlation between the EEG-amplitudes and the NCI z-scores to be expected. For the NCI effects to be measurable by means of magnetic resonance it is necessary that the condition of a collinear orientation of the direction of B and the spin-locking axes is fulfilled for the whole brain. This is to some degree fulfilled by changing the phase of the SL-pulse in steps of 50° (phase-cycling).

*Experimental Results*

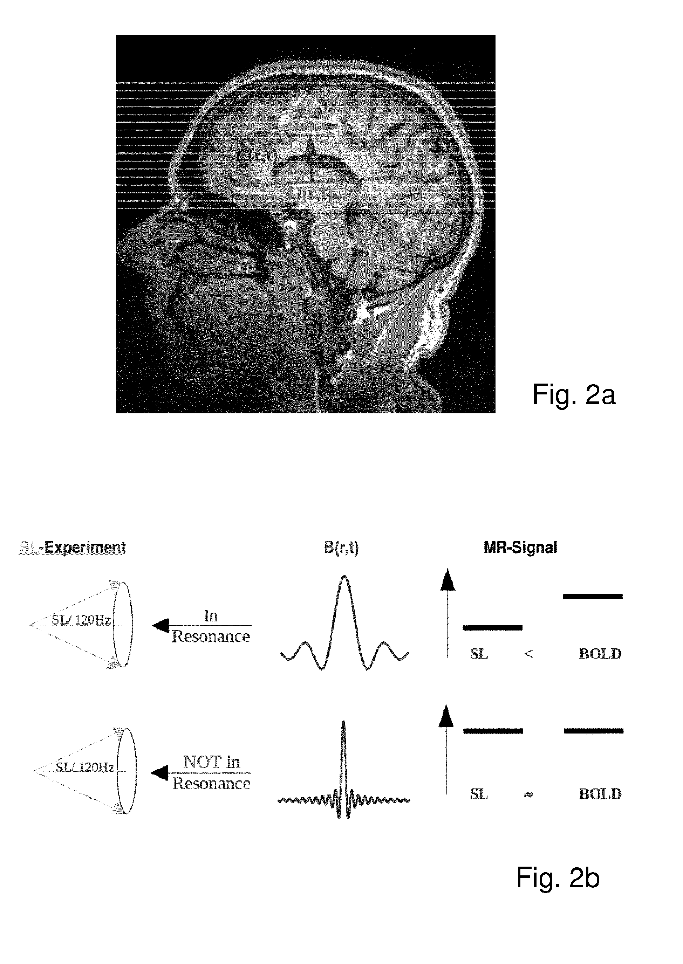

The present method locks most of the spins within the whole brain at a frequency of 120 Hertz. If there are any magnetic pulses (B(r)) collinear with the spin-locking axes, the resonance condition is fulfilled and typical pre-saturation of magnetization occurs within the period of the spin-locking part of the sequence. Its related signal is lower than the signal of the BOLD part obtained with the GRE part only due to the pre-saturation. If the B-pulse is high-frequency modulated due to effects coming from epileptic events, the resonance condition is not fulfilled and the spin-locked related signal is similar to the BOLD signal. Accordingly the difference signal (SL with GRE *minus* GRE only) is small. This in fact gives us the necessary image contrast. For a graphical representation of these theoretical relations, see Fig. 2.

A simulation of the entire sequence code (Fig. 3)without external influences such as brain currents, tissue distribution, coil peculiarities etc. showed that no essential NCI effects are caused by the sequence itself, i.e. no stimulated or indirect echoes are visible. The prominent feature in the signal trace is the main echo.

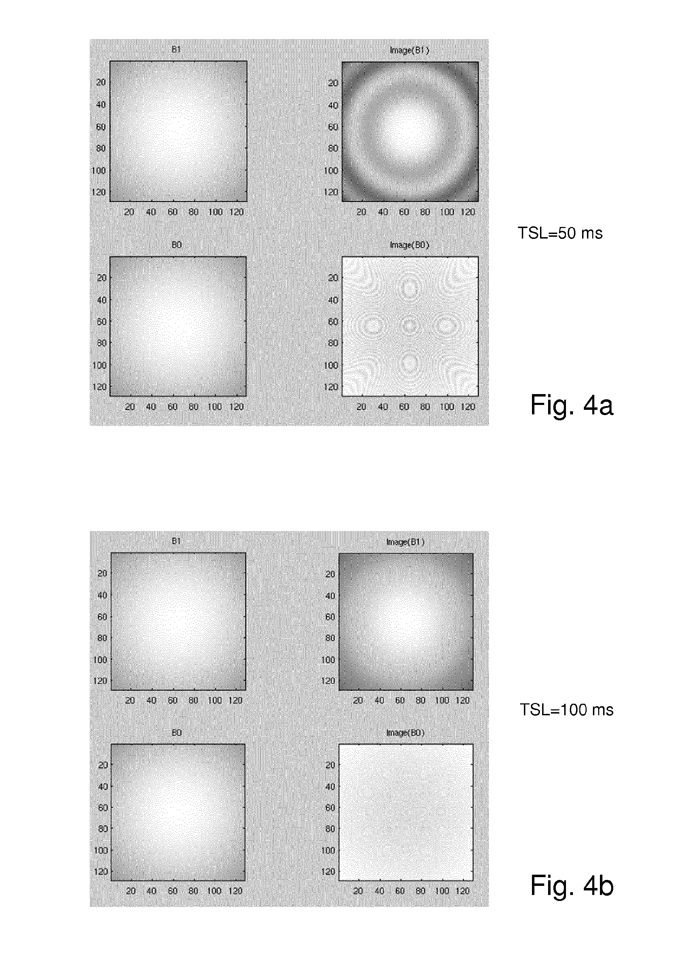

The effects of B1 inhomogeneity leading to ringing artifacts are more pronounced for a TSL of 50 ms compared to 100 ms (see Fig. 4a) vs. Fig. 4b)). So in order to optimally detect the pure NCI effects we switched to the TSL = 100 ms pulses during the study. The main NCI effects on the z-score maps are observed near the centers of epileptogenic events.

The maximal z-scores range between 8.6 and 9.9 whereas the functional overlays correspond to at least 60% of the maximal z-score. This means that the spin locking resonance condition seems not to be optimally fulfilled in these regions - the neuronal oscillation frequency within the lesion region is shifted with respect to the spin locking frequency - so that on the spin-locked image and the BOLD image the signal is more similar and the subtraction provides a more reduced value compared to the healthy brain tissue (see Fig. 2). At first glance one might think that the causes for this frequency shift are simply B1 inhomogeneities, but B1 effects could be excluded in most cases by choosing the optimal SL pulse length of TSL = 100 ms and the special phase-alternating pulse splitting.

So it appears that the shift out of the spin-locking frequency band is mainly caused by interictal high-frequency oscillations clearly greater than 120 Hz, e.g. 500 Hz, which are described in the work of Urrestarazu et al. (Urrestarazu et al., 2007).

An important difference to other approaches in literature is due to the additional phase stepping included in the SL sequence and to the adopted difference scheme acquired within one sequence. Both effects, the phase stepping (influence of more dipole orientations) and the approach to detect the difference between resonant and non-resonant (frequency shifted) effects - and not so much the SL related saturation effects itself - explain the achieved results.

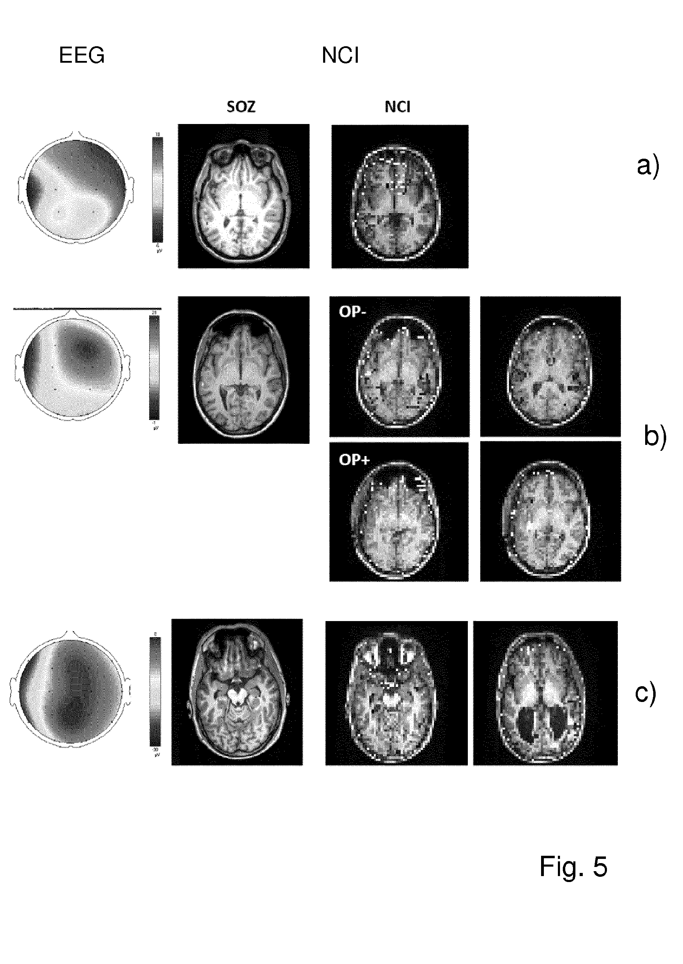

Fig. 5 shows a comparison of EEG and NCI data obtained with 3 patients a), b) and c). Patient a) had structural non-lesional lateral temporal lobe epilepsy, Patient b) had structural lesional lateral temporal lobe epilepsy, and Patient c) had structural epilepsy with mesial temporal lope sclerosis and hippocampal atrophy. The first column from left shows the EEG field distributions, the other columns (NCI) show images obtained with the present method. The second column from left (SOZ) shows the localization of the seizure onset zone (SOZ) as determined by visual analysis of EEG data and multimodal image coregistration of pre-implantation structural MRI and post-implantation CT. The third and (where present) fourth columns from left show further slices with the most important NCI effects. For patient b), these further slices are shown before (OP-) and after (OP+) surgery. The peak locations of the NCI effects in all three cases, marked as the z-scores above 60% of the maximum, correlate with the EEG field distribution and - most importantly - with the seizure-onset zone as determined with intracranial electroencephalography (green markers indicate electrode positions). Moreover, the NCI-effects, visible e.g. before resective surgery (Fig. 5b / OP-) disappeared after successful intervention (Fig. 5b / OP+).

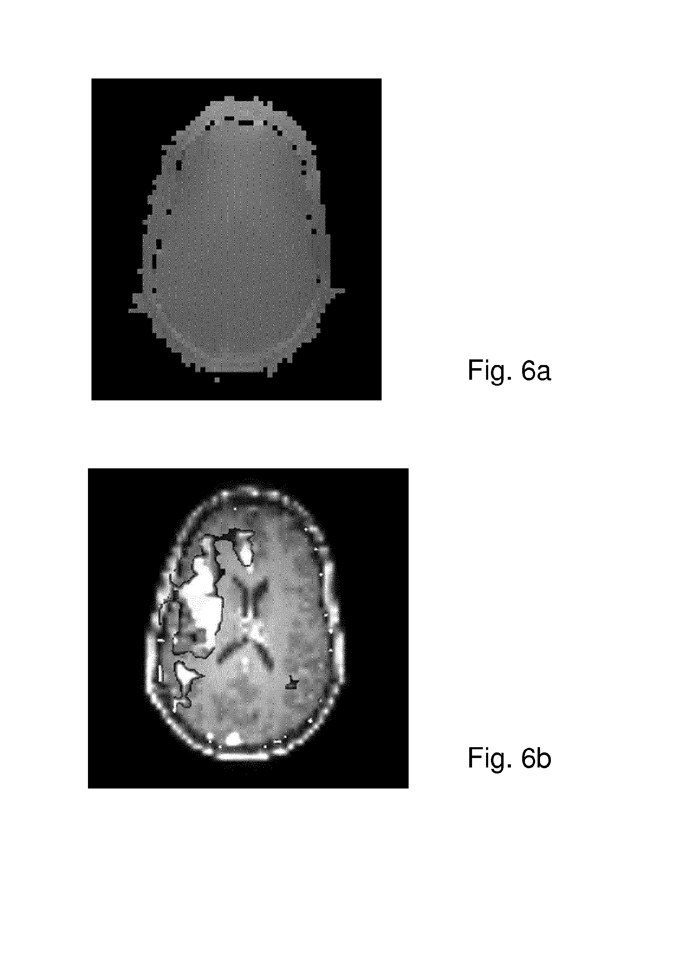

Readout related T2* effects are minimized by the subtraction of SL-images and BOLD images because the readout period of the sequence is the same for both acquisitions. Furthermore, the NCI effects are not related to general T2* or phase effects, as shown in Fig. 6.

In order to test the influence of BOLD effects we performed an NCI experiment with two different locking frequencies (120 and 500 Hz) - if the NCI effects were purely BOLD related there should be no difference between the two experiments because the change of the locking frequency only influences the SL-preparation. The pre-selection of high-frequency oscillations (HFO) in the range of 120 Hz was set empirically since we observed no SL effects at ultra-high HFOs (500 Hz) in our experiment.

*References*

Abragam, 1961 Abragam, A., 1961. Principles of Nuclear Magnetism. Oxford University Press.

Bandettini et al., 2005 Bandettini, P.A., Petridou, N., Bodurka, J., 2005. Direct detection of neuronal activity with MRI: fantasy, possibility, or reality? Appl. Magn. Reson 29.

Bloch and Siegert, 1940 Bloch, F., Siegert, A., 1940. Magnetic resonance for nonrotating fields. Phys. Rev. 57, 522.

Bodurka and Bandettini, 2002 Bodurka, J., Bandettini, P.A., 2002. Toward direct mapping of neuronal activity: MRI detection of ultraweak, transient magnetic field changes. Magn. Reson. Med. 47, 1052-1058.

Borthakur et al., 2006 Borthakur, A., Hulvershorn, J., Gualtieri, E., Wheaton, A.J., Charagundla, S., Elliott, M.A., Reddy, R., 2006. A pulse sequence for rapid in vivo spin-locked MRI. J. Magn. Reson.Imaging 23, 591-596.

Chow et al., 2006 Chow, L.S., Cook, G.G., Whitby, E., Paley, M.N., 2006. Investigating direct detection of axon firing in the adult human optic nerve using MRI. NeuroImage 30,835-846.

Chu et al., 2004 Chu, R., de Zwart, J.A., van Gelderen, P., Fukunaga, M., Kellman, P., Holroyd, T., Duyn, J.H.,2004. Hunting for neuronal currents: absence of rapid MRI signal changes during visual-evoked response. Neu-roImage 23, 1059-1067.

Konn et al., 2003 Konn, D., Gowland, P., Bowtell, R., 2003. MRI detection of weak magnetic fields due to an extended current dipole in a conducting sphere: a model for direct detection of neuronal currents in the brain. Magn. Reson. Med. 50, 40-49.

Kraus, 2006 Kraus Jr., R.H., 2006. Low field SQUID MRI. Workshop on Multimodal Functional Neuroimaging. Cortona, Italy.

Kraus et al., 2008 Kraus Jr., R.H., Volegov, P., Matlachov, A., Espy, M., 2008. Toward direct neural current imaging by resonant mechanisms at ultra-low field. NeuroImage 39, 310-317.

Matlachov et al., 2004 Matlachov, A.N., Volegov, P.L., Espy, M.A., George, J.S., Kraus Jr., R.H., 2004. SQUID detected NMR in microtesla magnetic fields. J. Magn. Reson. 170, 1-7.

Parkes et al., 2007 Parkes, L.M., de Lange, F.P., Fries, P., Toni, I., Norris, D.G., 2007. Inability to directly detect magnetic field changes associated with neuronal activity. Magn. Reson. Med. 57,411-416.

Redfield, 1955 Redfield, A.G., 1955. Nuclear magnetic resonance saturation and rotary saturation in solids.Phys. Rev. 98, 1787.

Urrestarazu et al., 2007 Elena Urrestarazu, Rahul Chander, Francois Dubeau and Jean Gotman. Interictal high-frequency oscillations (100-500Hz) in the intracerebral EEG of epileptic patients. Brain (2007), 130, 2354-2366.

Volegov et al., 2004Volegov, P., Matlachov, A.N., Espy, M.A., George, J.S., Kraus Jr., R.H., 2004. Simultaneous magnetoencephalography and SQUID detected nuclear MR in microTesla magnetic fields. Magn. Reson. Med. 52, 467-470.

Xiong et al.,2003 Xiong, J., Fox, P.T., Gao, J.H., 2003. Directly mapping magnetic field effects of neuronal activity by magnetic resonance imaging. Hum. Brain Mapp. 20, 41-49.

**Claims**

1. A magnetic resonance (MR) imaging method, particularly for detecting neuronal activity in a subject's brain, the method comprising:

   a) performing a first MR imaging sequence part with a MR imaging system having a static magnetic field in z-direction, said first sequence part comprising:

   - a preparatory spin-locking pulse sequence part comprising an initial spin-tipping pulse followed by a spin-lock pulse followed by a final spin-tipping pulse, followed by spoiler gradient pulses for removing any remaining transverse magnetization;
   wherein said initial spin-tipping pulse is a 90° pulse in y direction and said final spin-tipping pulse is a 90° pulse in -y direction, or vice versa;
   wherein said spin-lock pulse is a rectangular pulse in x direction consisting of two phase-alternating halves and having an amplitude $B_{1lock}$, a spin-lock pulse duration TSL and an initial spin-lock phase $\varphi_{1SL}$;
   - an acquisition pulse sequence part to acquire first MR data, wherein said acquisition pulse sequence part is an echo planar imaging sequence;

   b) performing a second MR imaging sequence part with said MR imaging system, said second sequence part comprising said acquisition pulse sequence part to acquire second MR data; and

   c) repeating steps a) and b) for a predetermined number of instances, wherein the initial spin-lock phase $\varphi_{1SL}$ is increased by a predetermined phase increment $\Delta\varphi_{1SL}$, thereby obtaining multiple sets of first and second MR data.

2. The method according to claim 1, wherein said echo planar imaging sequence is a gradient echo planar imaging sequence.

3. The method according to claim 1 or 2, wherein said spin-lock pulse has an amplitude $B_{1lock}$ corresponding to a Larmor frequency of 100 to 200 Hz, preferably about 120 Hz.

4. The method according to one of claims 1 to 3, wherein the spin-lock pulse duration TSL is 50 to 200 ms, preferably about 100 ms.

5. The method according to one of claims 1 to 4, wherein the phase increment $\Delta\varphi_{1SL}$ is 40° to 60°, preferably about 50°.

6. A method of detecting neuronal activity in a subject's brain, comprising:

   a) carrying out the MR imaging method according to one of claims 1 to 5 with the subject's brain positioned in the MR imaging system;
   b) obtaining difference MR data by subtracting said second MR data from said first MR data; and
   c) generating an image from said difference MR data, wherein zones of comparatively high brightness are indicative of high-frequency neuronal currents.

7. The method according to claim 6, wherein said image is represented as a z-score map obtained by a raw image reconstructed from said difference MR data.

8. The method according to claim 7, wherein said z-score map includes a threshold of 50 to 70%, preferably about 60%.

9. A computer program product comprising program code means stored on a computer readable medium for causing a computer system to control the execution of the method according to any one of claims 6 to 8 when the computer program product is run on the computer system.

Fig. 1

Fig. 3

Fig. 2a

Fig. 2b

TSL=50 ms

Fig. 4a

TSL=100 ms

Fig. 4b

Fig. 5

Fig. 6a

Fig. 6b

**EUROPEAN SEARCH REPORT**

Application Number

EP 14 17 7733

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | N. W. HALPERN-MANNERS ET AL: "Magnetic resonance imaging of oscillating electrical currents", PROCEEDINGS OF THE NATIONAL ACADEMY OF SCIENCES, vol. 107, no. 19, 11 May 2010 (2010-05-11), pages 8519-8524, XP055137425, ISSN: 0027-8424, DOI: 10.1073/pnas.1003146107 * the whole document * | 1-9 | INV. G01R33/20 A61B5/055 G01R33/48 |
| X | WITZEL T ET AL: "Stimulus-induced Rotary Saturation (SIRS): A potential method for the detection of neuronal currents with MRI", NEUROIMAGE, ACADEMIC PRESS, ORLANDO, FL, US, vol. 42, no. 4, 1 October 2008 (2008-10-01), pages 1357-1365, XP024530155, ISSN: 1053-8119, DOI: 10.1016/J.NEUROIMAGE.2008.05.010 [retrieved on 2008-05-20] * the whole document * | 1-9 | |
| A | FRANCESCO DE LUCA: "Direct fMRI by random spin-lock along the neural field", MAGNETIC RESONANCE IMAGING, ELSEVIER SCIENCE, TARRYTOWN, NY, US, vol. 29, no. 7, 3 April 2011 (2011-04-03), pages 951-957, XP028266025, ISSN: 0730-725X, DOI: 10.1016/J.MRI.2011.04.003 [retrieved on 2011-04-13] * the whole document * | 1-9 | |

TECHNICAL FIELDS SEARCHED (IPC)

G01R
A61B

-/--

The present search report has been drawn up for all claims

| Place of search | Date of completion of the search | Examiner |
|---|---|---|
| Munich | 1 September 2014 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

# EUROPEAN SEARCH REPORT

Application Number

EP 14 17 7733

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | ARIJITT BORTHAKUR ET AL: "A pulse sequence for rapid in vivo spin-locked MRI", JOURNAL OF MAGNETIC RESONANCE IMAGING, vol. 23, no. 4, 1 January 2006 (2006-01-01), pages 591-596, XP055137412, ISSN: 1053-1807, DOI: 10.1002/jmri.20537 * the whole document * | 1-9 | |
| A | PADMAVATHI SUNDARAM ET AL: "Fast human brain magnetic resonance responses associated with epileptiform spikes", MAGNETIC RESONANCE IN MEDICINE, vol. 64, no. 6, 30 August 2010 (2010-08-30), pages 1728-1738, XP055137414, ISSN: 0740-3194, DOI: 10.1002/mrm.22561 * the whole document * | 1-9 | |
| A | RODIONOV R ET AL: "Looking for neuronal currents using MRI: An EEG-fMRI investigation of fast MR signal changes time-locked to frequent focal epileptic discharges", NEUROIMAGE, ACADEMIC PRESS, ORLANDO, FL, US, vol. 50, no. 3, 15 April 2010 (2010-04-15), pages 1109-1117, XP026926034, ISSN: 1053-8119, DOI: 10.1016/J.NEUROIMAGE.2009.12.076 [retrieved on 2010-02-25] * the whole document * | 1-9 | TECHNICAL FIELDS SEARCHED (IPC) |

The present search report has been drawn up for all claims

| Place of search | Date of completion of the search | Examiner |
|---|---|---|
| Munich | 1 September 2014 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ABRAGAM, A.** Principles of Nuclear Magnetism. Oxford University Press, 1961
- **BANDETTINI, P.A. ; PETRIDOU, N. ; BODURKA, J.** Direct detection of neuronal activity with MRI: fantasy, possibility, or reality?. *Appl. Magn. Reson,* 2005, 29
- **BLOCH, F. ; SIEGERT, A.** Magnetic resonance for nonrotating fields. *Phys. Rev.,* 1940, vol. 57, 522
- **BODURKA, J. ; BANDETTINI, P.A.** Toward direct mapping of neuronal activity: MRI detection of ultraweak, transient magnetic field changes. *Magn. Reson. Med.,* 2002, vol. 47, 1052-1058
- **BORTHAKUR, A. ; HULVERSHORN, J. ; GUALTIERI, E. ; WHEATON, A.J. ; CHARAGUNDLA, S. ; ELLIOTT, M.A. ; REDDY, R.** A pulse sequence for rapid in vivo spin-locked MRI. *J. Magn. Reson.Imaging,* 2006, vol. 23, 591-596
- **CHOW, L.S. ; COOK, G.G. ; WHITBY, E. ; PALEY, M.N.** Investigating direct detection of axon firing in the adult human optic nerve using MRI. *NeuroImage,* 2006, vol. 30, 835-846
- **CHU, R. ; DE ZWART, J.A. ; VAN GELDEREN, P. ; FUKUNAGA, M. ; KELLMAN, P. ; HOLROYD, T. ; DUYN, J.H.** Hunting for neuronal currents: absence of rapid MRI signal changes during visual-evoked response. *Neu-rolmage,* 2004, vol. 23, 1059-1067
- **KONN, D. ; GOWLAND, P. ; BOWTELL, R.** MRI detection of weak magnetic fields due to an extended current dipole in a conducting sphere: a model for direct detection of neuronal currents in the brain. *Magn. Reson. Med.,* 2003, vol. 50, 40-49
- **KRAUS JR., R.H.** Low field SQUID MRI. *Workshop on Multimodal Functional Neuroimaging,* 2006
- **KRAUS JR., R.H. ; VOLEGOV, P. ; MATLACHOV, A. ; ESPY, M.** Toward direct neural current imaging by resonant mechanisms at ultra-low field. *NeuroImage,* 2008, vol. 39, 310-317
- **MATLACHOV, A.N. ; VOLEGOV, P.L. ; ESPY, M.A. ; GEORGE, J.S. ; KRAUS JR., R.H.** SQUID detected NMR in microtesla magnetic fields. *J. Magn. Reson.,* 2004, vol. 170, 1-7
- **PARKES, L.M. ; DE LANGE, F.P. ; FRIES, P. ; TONI, I. ; NORRIS, D.G.** Inability to directly detect magnetic field changes associated with neuronal activity. *Magn. Reson. Med.,* 2007, vol. 57, 411-416
- **REDFIELD, A.G.** Nuclear magnetic resonance saturation and rotary saturation in solids. *Phys. Rev.,* 1955, vol. 98, 1787
- **ELENA URRESTARAZU ; RAHUL CHANDER ; FRANCOIS DUBEAU ; JEAN GOTMAN.** Interictal high-frequency oscillations (100-500Hz) in the intracerebral EEG of epileptic patients. *Brain,* 2007, vol. 130, 2354-2366
- **VOLEGOV, P. ; MATLACHOV, A.N. ; ESPY, M.A. ; GEORGE, J.S. ; KRAUS JR., R.H.** Simultaneous magnetoencephalography and SQUID detected nuclear MR in microTesla magnetic fields. *Magn. Reson. Med.,* 2004, vol. 52, 467-470
- **XIONG, J. ; FOX, P.T. ; GAO, J.H.** Directly mapping magnetic field effects of neuronal activity by magnetic resonance imaging. *Hum. Brain Mapp.,* 2003, vol. 20, 41-49